# EUROPEAN PATENT APPLICATION

(11) **EP 4 147 712 A1**
(43) Date of publication of application: **15.03.2023**
(21) Application number: 21196390.5
(22) Date of filing: 13.09.2021
(51) Int. Cl.: A61K 39/00

(54) **METHOD TO GENERATE A DOUBLE-STRANDED DNA POOL ENCODING NEOANTIGENS OF A TUMOR OF A PATIENT**

(71) Applicant: OncoDNA, 6041 Gosselies (BE)
(72) Inventor: Detiffe, Jean-Pol, 6500 Beaumont (BE); Van Huffel, Christophe, 1332 Rixensart (BE); Marcaillou, Charles, 94230 Cachan (FR)
(74) Representative: Calysta NV

(57) **Abstract**

A synthetic DNA molecule comprising one segment encoding a tumor neoantigen or an epitope from an infectious agent under the control of a promoter for the transcription into a corresponding RNA molecule and a segment for the translation of the said translated RNA molecule into a peptide.

## Description

### Technical domain

The present disclosure concerns the use of information extracted from the genome of a patient to generate tumor neoantigens. In particular, the discovery of a synthetic DNA molecule comprising one segment encoding a tumor neoantigen or an epitope from an infectious agent. Such synthetic DNA molecule may be used as a template in a RNA vaccine, e.g. a personalized vaccine comprising the tumor neoantigen of a tumor of a patient or a vaccine comprising the epitope from an infectious agent.

More specifically, but not exclusively, the present disclosure refers to a process for the production of a DNA molecule able to be transcribed into a RNA vaccine.

The present disclosure also relates to a pool of the DNA molecules according to the present invention.

The present disclosure also concerns a process for the generation of a RNA vaccine towards a cancer affecting a patient comprising the steps of generating the DNA molecule according to the present invention or the pool according to the present invention.

The present disclosure also relates to the vaccine obtainable by the process for the generation of a RNA vaccine according to the present invention.

### State of the art

Document US2015038373A1 provides a method for de novo gene synthesis. In particular, the document provides methods, devices and systems that can quickly synthesize large gene libraries or relatively longer oligonucleotide fragments efficiently with less error. The substrate used for de novo gene synthesis is silicon.

The document WO2016126987A1 provides methods for nucleic acid assembly, comprising: providing a predetermined nucleic acid sequence; providing a plurality of precursor double-stranded nucleic acid fragments, each precursor double-stranded nucleic acid fragment having two strands, wherein each of the two strands comprises a sticky end sequence of 5' -A (N_{X} ) T-3' or 5'-G (Nx ) C-3', wherein N is a nucleotide, wherein x is the number of nucleotides between nucleotides A and T or between G and C, and wherein x is 1 to 10, and wherein no more than two precursor double-stranded nucleic acid fragments comprise the same sticky end sequence.

The document WO2016172377A1 provides devices for synthesizing oligonucleic acids, comprising: a plate; a main channel, wherein the main channel extends vertically into the plate from an opening on a top side of the plate, and wherein the main channel has a width of 0.5 to 2 mm; and a plurality of microchannels connected to the main channel, wherein each microchannel of the plurality of microchannels extends vertically from an opening on a bottom side of the plate into the main channel.

The document US2020222875 provides systems, methods, and compositions for the efficient de novo synthesis of highly accurate and uniform polynucleotide libraries. In particular, the document provides methods for polynucleotide synthesis, comprising: a) providing a structure comprising a surface; b) coupling at least one nucleoside to a polynucleotide attached to the surface; c) depositing an oxidizing solution on the surface; d) depositing a wash solvent on the surface, wherein the wash solvent comprises a ketone, an ester, an ether, a hydrocarbon, or a functional equivalent thereof; and e) repeating steps b-d to synthesize a plurality of polynucleotides.

The document EP3576751 provides a ribonucleic acid (RNA) cancer vaccine that can safely direct the body's cellular machinery to produce nearly any cancer protein or fragment thereof of interest. This document exemplified a cancer vaccine having 20 epitopes or 52 epitopes. The document describes a mRNA cancer vaccine having an open reading frame encoding a concatemer of epitopes.

Unfortunately, although these documents describe the merits of their technology, currently de novo synthesis of DNA molecule, preferably a long DNA molecule having a length superior to 200 nucleotides, encoding for a tumor specific antigen (neoantigen) identified from the tumor of the patient or for the epitope from an infectious agent does not exist. In particular, no method exists to produce said DNA molecule in pool permitting the generation, in the pool, of a plurality of DNA molecules encoding a plurality of different tumor neoantigens or epitopes from an infectious disease. Indeed, a disease, e.g. a cancer, may be characterized by numerous disease-specific antigens. The production of an efficient personalized RNA vaccine rests on the development of a vaccine comprising a sufficiently large number of disease-specific, e.g. tumor-specific, antigens able to elicit a proper immunological response in the patient. Currently, the efficacy of personalized vaccine is not high, in part due to the fact that the number of personalized epitopes or neoantigens that can be incorporated into the vaccine is limited. Indeed, personalized vaccines from the state of the art rest on DNA or RNA encoding an open reading frame having a concatemer of epitopes. The size of the DNA or RNA is limited by the maximum size of its corresponding plasmid. Since the size is limited, it cannot incorporate a very large number of different epitopes needed to elicit a large immunological response characterized by the production of specific T cells targeting the different epitopes. State of the art also mentioned de novo production of DNA fragments, comprising concatemeric epitopes, that can be used as a template into a vaccine but such de novo production is limited by the synthesis of short DNA fragments having a length smallerthan or equal to 200 nucleotides, wherein these short DNA fragments are further assembled by multiple ligations. Thus, another important limitation in the current methods from the state of the art is the presence of multiple ligation steps which can introduce errors in the resulting DNA fragments.

### Summary of the invention

There is therefore a need to dispose of a synthetic DNA molecule comprising one segment encoding a tumor neoantigen or an epitope from an infectious agent wherein, preferably, the DNA molecule having a length equal or superior to 200 nucleotides and can be produced in a pool of DNA molecules without limitation in the number of different DNA molecules present in the pool.

According to the present invention, a synthetic DNA molecule comprising one segment encoding a tumor neoantigen or an epitope from an infectious agent under the control of a promoter for the transcription into a corresponding RNA molecule and a segment for the translation of the said translated RNA molecule into a peptide is provided.

According to the present invention, a (complementary) DNA molecule comprising (i) a segment encoding a (second) portion of a peptide for addressing an antigen to MHC molecules, (ii) a segment for increasing the stability of a transcribed RNA or for increasing the translation of the transcribed RNA into a protein, and (iii) a segment encoding a poly A tail is also provided. As it will be further described in the detailed description, said (complementary) DNA molecule according to the present invention will be used as a reverse primer allowing for the generation of a plurality of double-stranded DNA molecules in a pool using only one reverse primer.

The present invention also relates to a process for the production of the DNA molecule according to the present invention, comprising the steps of performing the chemical synthesis of a DNA molecule encoding a tumor neoantigen under the control of a promoter.

Another object of the present invention is a pool of the DNA molecules according to the present invention or the pool obtainable by the process according to the present invention, the said pool comprising a plurality of different tumor neoantigens or epitopes from an infectious agent. Indeed, a pool of the DNA molecules according to the present invention comprising a plurality of different tumor neoantigens or of different epitopes from an infectious agent is not limited in the number of different neoantigens or epitopes present in the pool. As a consequence, the pool of DNA molecules according to the present invention may serve as a template for a personalized vaccine of the patient that can incorporate an unlimited number of neoantigens of the patient and can thus elicits an optimal immunological response ensuring a high efficacy of the personalized vaccine.

The present invention also refers to a process for the generation of a RNA vaccine towards a cancer affecting a patient comprising the steps of generating the DNA molecule according to the present invention or the pool according to the present invention and of performing in vitro transcription of the DNA molecule(s) into RNA molecule(s).

The present invention also refers to the vaccine obtainable by the process according to the present invention.

Dependent claims refer to other advantageous embodiments.
Figure 1 is a scheme of a DNA molecule according to the present invention comprising different fragments together with the corresponding transcribed RNA and translated polynucleotide.
Figure 2 illustrates different variations of the DNA molecule according to the present invention.
Figure 3 shows a DNA molecule according to the present invention together with an overlapping region of two single-stranded DNA molecules.
Figure 4 shows a scheme illustrating a partial overlap hybridization between a first and a second single stranded DNA molecule.
Figure 5 illustrates a pool of DNA molecules according to the present invention.
Figure 6 illustrates an example of a sequence of the DNA molecule according to the present invention.
Figure 7 illustrates an example of the sequence of the DNA molecule and the corresponding protein sequence according to the present invention.
Figure 8 illustrates an example of the sequence of the DNA molecule and the corresponding protein sequence according to the present invention together with the forward and reverse primers.

In the drawings, the same reference numbers have been allocated to the same or analogue element.

Other characteristics and advantages of the present invention will be derived from the non-limitative following description, and by making reference to the drawings and the examples.

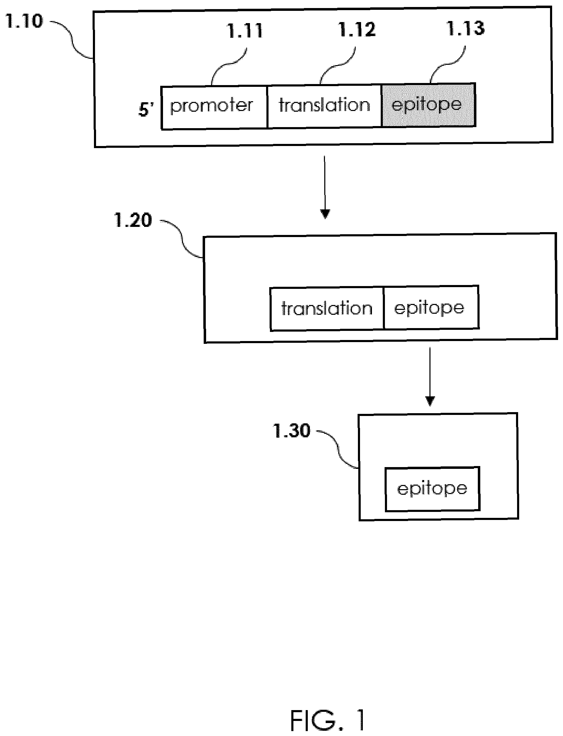

Figure 1 illustrates a scheme showing a synthetic DNA molecule 1.10 according to the present invention comprising one segment 1.13 encoding a tumor neoantigen or an epitope from an infectious agent under the control of a promoter 1.11 for the transcription into a corresponding RNA molecule 1.20 and a segment for the translation 1.12 of the said transcribed RNA molecule 1.20 into a peptide 1.30.

Preferably, the DNA molecule 1.10 is synthetized as a single-stranded DNA molecule.

Preferably, the segment 1.13 encoding a tumor neoantigen or an epitope from an infectious agent is a sequence comprising between 21 and 504 nucleotides, preferably between 51 and 201 nucleotides, more preferably between 63 and 84 nucleotides, favorably of 81 nucleotides, wherein the segment 1.13 encodes favorably for a tumor-specific antigen of a tumor of a patient.

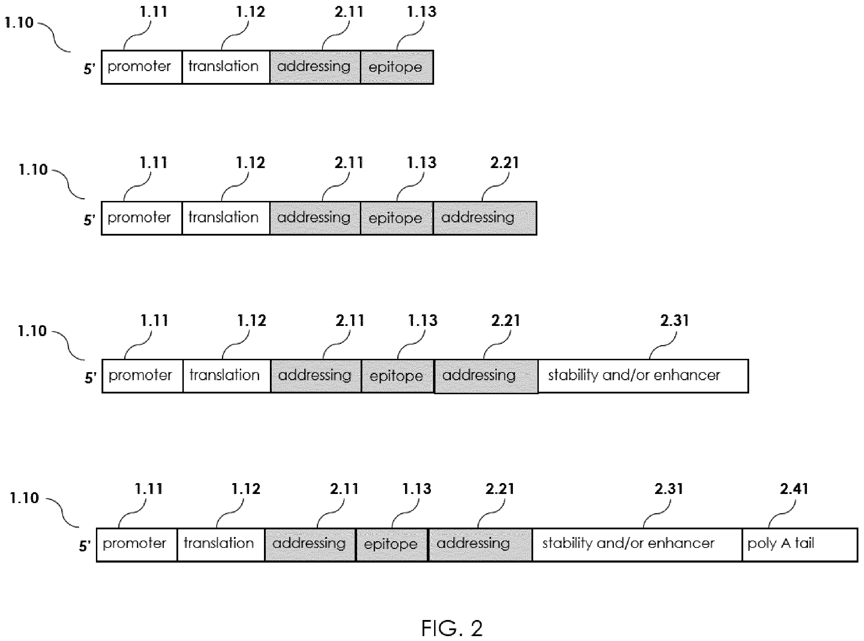

Figure 2 indicates that the DNA molecule 1.10 according to the present invention preferably further comprises a segment 2.11 encoding a sequence for addressing and/or stabilizing and/or targeting and/or trafficking the tumor neoantigen or the epitope from an infectious agent into a vesicular region or at the cell surface. Preferably, the DNA molecule 1.10 comprises a segment 2.21 encoding a sequence for addressing the encoded tumor neoantigen or the epitope from an infectious agent to MHC molecules. Advantageously, the DNA molecule 1.10 further comprising a segment 2.31 encoding a translation enhancer. Preferably, the DNA molecule 1.10 comprises a 3'-UTR and/ora 3' Poly A tail segment(s) 2.41 and/or a 5'-UTR. Advantageously, the DNA molecule according to the present invention is not limited to the DNA molecules illustrated in Figs. 1 and 2. Indeed, the DNA molecule according to the present invention comprising at least the promoter 1.11, the segment for the translation 1.12 and the segment 1.13 encoding a tumor neoantigen or an epitope from an infectious agent. Preferably, the DNA molecule may further comprise the segment 2.11 encoding a sequence for stabilizing the tumor neoantigen or the epitope from an infectious agent and/or targeting and/or trafficking it in a vesicular region and/or at the cell surface, and/or the segment 2.21 encoding a sequence for addressing the encoded tumor neoantigen or the epitope from an infectious agent to MHC molecules, and/or the segment 2.31 encoding a translation enhancer and/or the 3'-UTR and/or a 3' Poly A tail segment(s) 2.41 and/or the 5'-UTR, or any combination of the segments 2.11 and/or 2.21 and/or 2.31 and/or 2.41. According to the present invention, the 5' to 3' succession order of the different segments 1.11 and/or 1.12 and/or 2.11 and/or 1.13 and/or 2.21 and/or 2.31 and/or 2.41 of the DNA molecules may vary and is not limited to the succession order illustrated in the DNA molecule shown in Figs. 1 to 3. Advantageously, the inventors found that each DNA segment 2.11 and/or 2.21 and/or 2.31 and/or 2.41 added to the DNA molecule 1.10 increases each, by about 5-fold, the peptide expression of the tumor neoantigen or the epitope from an infectious agent 1.13.

Advantageously, a synthetic DNA molecule comprising one segment encoding a tumor neoantigen or an epitope from an infectious agent under the control of a promoter for the transcription into a corresponding RNA molecule and a segment for the translation of the said transcribed RNA molecule into a peptide further comprises a segment encoding a sequence for stabilizing and/or targeting and/or trafficking the tumor neoantigen or the epitope from an infectious agent in a vesicular region, and preferably further comprises a segment encoding a sequence for addressing the encoded tumor neoantigen or the epitope from an infectious agent to MHC molecules, a segment encoding a translation enhancer, and a 3'-UTR and/or a 3' Poly A tail segment(s) and/or a 5'-UTR.

Alternatively, a synthetic DNA molecule comprising one segment encoding a tumor neoantigen or an epitope from an infectious agent under the control of a promoter for the transcription into a corresponding RNA molecule and a segment for the translation of the said transcribed RNA molecule into a peptide further comprises a segment encoding a sequence for addressing the encoded tumor neoantigen or the epitope from an infectious agent to MHC molecules, and preferably, further comprises a segment encoding a sequence for stabilizing and/or targeting and/or trafficking the tumor neoantigen or the epitope from an infectious agent in a vesicular region, a segment encoding a translation enhancer, and a 3'-UTR and/or a 3' Poly A tail segment(s) and/or a 5'-UTR.

Alternatively, a synthetic DNA molecule comprising one segment encoding a tumor neoantigen or an epitope from an infectious agent under the control of a promoter for the transcription into a corresponding RNA molecule and a segment for the translation of the said transcribed RNA molecule into a peptide further comprises a segment encoding a translation enhancer, and preferably, further comprises a sequence for stabilizing and/or targeting and/or trafficking the tumor neoantigen or the epitope from an infectious agent in a vesicular region, a segment encoding a sequence for addressing the encoded tumor neoantigen or the epitope from an infectious agent to MHC molecules and a 3'-UTR and/or a 3' Poly A tail segment(s) and/or a 5'-UTR.

Alternatively, a synthetic DNA molecule comprising one segment encoding a tumor neoantigen or an epitope from an infectious agent under the control of a promoter for the transcription into a corresponding RNA molecule and a segment for the translation of the said transcribed RNA molecule into a peptide further comprises a 3'-UTR and/or a 3' Poly A tail segment(s) and/or a 5'-UTR, and preferably, further comprises a sequence for stabilizing and/or targeting and/or trafficking the tumor neoantigen or the epitope from an infectious agent in a vesicular region, a sequence for addressing the encoded tumor neoantigen or the epitope from an infectious agent to MHC molecules, and a segment encoding a translation enhancer.

Preferably, the promoter 1.11 is a bacteriophage RNA polymerase promoter. More preferably, the promoter 1.11 belongs to a group of promoters comprising a promoter sequence of T7 RNA polymerase, of SP6 RNA polymerase, of T3 RNA polymerase, of Syn5 RNA polymerase, of KP34 RNA polymerase. Even more preferably, according to the present invention, the promoter sequence of T7 RNA polymerase is a binding sequence for T7 RNA polymerase having an identity of at least 95% towards SEQ ID NO :1 over its whole sequence length, the promoter sequence of SP6 RNA polymerase is a binding sequence for SP6 RNA polymerase having an identity of at least 95% towards SEQ ID NO :73 over its whole sequence length, the promoter sequence of T3 RNA polymerase is a binding sequence for T3 RNA polymerase having an identity of at least 95% towards SEQ ID NO :74 over its whole sequence length, the promoter sequence of Syn5 RNA polymerase is a binding sequence for Syn5 RNA polymerase having an identity of at least 95% towards SEQ ID NO :75 over its whole sequence length, the promoter sequence of KP34 RNA polymerase is a binding sequence for KP34 RNA polymerase having an identity of at least 95% towards SEQ ID NO :76 (strong promotor 1) over its whole sequence length or towards SEQ ID NO :77 (strong promotor 2) over its whole sequence length or towards SEQ ID NO :78 (weak promotor) over its whole sequence length. Favorably, the promoter 1.11 according to the present invention is the promoter sequence of T7 RNA polymerase having an identity of at least 95% towards SEQ ID NO :1 over its whole sequence length.

Advantageously, the segment for the translation 1.12 is a translation enhancer sequence located in a 5'UTR region (upstream of a coding region) of the DNA molecule 1.10. The coding region, coding part of the DNA molecule 1.10 is represented by the grey boxes in Figs. 1, 2 and 3. Preferably, the segment for the translation 1.12 is a translation enhancer/promoter sequence chosen from the group of translation enhancer/promoter sequence comprising Human Cytomegalovirus (CMV) enhancer, alpha or beta-globin enhancer, EF-1 alpha enhancer, simian virus 40 (SV40) enhancer, PGK1 promoter, HSD17B4 promoter, Ubiquitin C promoter, and beta-Actin promoter. More preferably, the segment for the translation 1.12 is a beta-globin translation enhancer sequence. Favorably, the beta-globin sequence has an identity of at least 95% towards SEQ ID NO :2 over its whole sequence length.

Preferably, the segment 2.11 encoding a sequence for addressing and/or stabilizing and/or targeting and/or trafficking the tumor neoantigen or the epitope from an infectious agent into a vesicular region is a signal sequence for endosomial processing, for example the signal sequence of a lysosome-associated membrane protein (LAMP1 or LAMP2; such as SEQ ID NO:3), possibly of dendritic cells (DC-LAMP) or a sequence for the addressing to vesicular compartments for coupling there to the MHC-I receptor (such as SEQ ID NO:5). Favorably, the signal sequence for endosomial processing wherein having favorably an identity of at least 95% towards SEQ ID NO :3 over its whole sequence length.

Preferably, the segment 2.21 encoding a sequence for addressing the encoded tumor neoantigen or the epitope from an infectious agent to MHC molecules is an immune processing for presentation to T lymphocytes. More preferably, the segment 2.21 is a dendritic cell lysosomal associated membrane glycoprotein signal sequence having, favorably, an identity of at least 95% towards SEQ ID NO:5 over its whole sequence length.

Advantageously, the segment 2.31 encoding a translation enhancer is a translation enhancer and/or a translation stability sequence preferentially located in a 3'UTR region (downstream of the coding region) of the DNA molecule 1.10. Preferably, the segment 2.31 is a beta-globin translation enhancer sequence, wherein the beta globin translation enhancer having, preferably, an identity of at least 95% towards SEQ ID NO:6 over its whole sequence length.

Preferably, the segment 2.41 is a 3' Poly A tail sequence of at least 68 nucleotides, preferably at least 100 nucleotides.

Preferably, the DNA molecule may further comprise 3' and/or 5' segments corresponding to conserved sequence elements (3' and/or 5' CSE segments). These 3' and/or 5' CSE segments are advantageously recognized by a RNA-dependent RNA polymerase (RdRp) allowing the RNA amplification of the transcribed DNA molecule.

Advantageously, the DNA molecule according to the present invention has a size comprised between 100 nucleotides per strand and about 2500 nucleotides per strand, preferably between 300 nucleotides per strand and about 800 nucleotides per strand, more preferably between about 400 nucleotides per strand and about 700 nucleotides per strand, still more preferably between about 500 (or 550) nucleotides per strand and about 650 (or 600) nucleotides per strand.

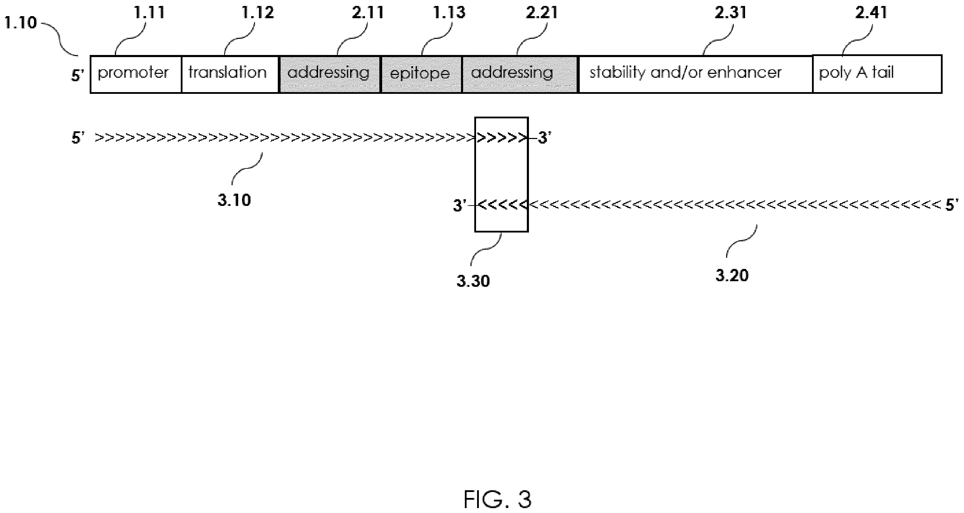

Figure 3 shows, according to the present invention, that a (complementary) DNA molecule 3.20 comprises (i) a segment encoding a (second) portion of the segment 2.21 of a peptide for addressing an antigen (the epitope 1.13) to MHC molecules, (ii) a segment 2.31 for increasing the stability of a transcribed RNA or for increasing the translation of the transcribed RNA into a protein, and (iii) a segment 2.41 encoding a poly A tail. Advantageously, the DNA molecule 3.20 according to the present invention, being in antisense, preferably for base pairing with a sense DNA molecule 3.10 comprising a segment 1.13 encoding an antigen, or an epitope, under the control of a promoter for the transcription into a corresponding RNA molecule, the segment 1.12 for its translation, the segment 2.11 encoding a sequence for addressing and/or stabilizing and/or targeting and/or trafficking the tumor neoantigen or the epitope from an infectious agent into a vesicular region and a segment encoding a (first) portion of the segment 2.21 of a peptide for the loading of an antigen for presentation to T lymphocytes, wherein the said first and second portion form the complete sequence 2.21 for the loading of an antigen for presentation to T lymphocytes and wherein the said first and second portions, being in sense and antisense, present an overlap sequence 3.30 allowing base pairing of the DNA molecule 3.10 with the DNA molecule 3.20.

Advantageously, the overlap sequence 3.30 is a continuous sequence of at least 5 nucleotides, preferably at least 10 nucleotides, more preferably at least 15 nucleotides, favorably at least 20 nucleotides and/or preferably of maximum 100 nucleotides, more preferably of maximum 75 nucleotides, favorably of maximum 40 nucleotides. Preferably, the overlap sequence 3.30 is located in a constant sequence region. According to the present invention, the constant sequence region refers to a sequence region present in every DNA molecule 1.10 according to the present invention.

Preferably, the overlap sequence is a sequence having an identity of at least 95% towards a sequence corresponding to nucleotides 30 to 52 within the SEQ ID NO :5 over its whole sequence length.

Preferably, a process for the production of the DNA molecule, according to the present invention, comprises the steps of performing a chemical synthesis of a DNA molecule encoding a tumor neoantigen under the control of a promoter.

Preferably, the chemical synthesis is a single-stranded oligonucleotides synthesis using a silicon-based DNA writing technology (i.e. US2018104664A1).

Preferably, the process according to the present invention comprises the steps of performing the synthesis of :
(i) a first single stranded DNA molecule 3.10 comprising a promoter 1.11 for the transcription in RNA, a segment 1.12 for its translation and a targeting sequence 2.11 for endosomial addressing fused in-frame with a tumor neoantigen or an epitope from an infectious agent 1.13 and a first portion of a genetic element 2.21 encoding a sequence for the loading of the encoded tumor neoantigen or the epitope from an infectious agent for presentation to T lymphocytes, and
(ii) a second single stranded DNA molecule 3.20 encoding a second portion of a genetic element 2.21 encoding a sequence for the loading of the encoded tumor neoantigen or the epitope from an infectious agent for presentation to T lymphocytes, wherein the second single stranded DNA molecule is antisense and wherein the said first and second portions form the complete sequence 2.21 for the loading of the tumor neoantigen or the epitope from an infectious agent for presentation to T lymphocytes and wherein the said first and second portions present an overlap 3.30 (of at least 15 consecutive nucleotides) allowing a specific base pairing,
the said process further comprising the step of hybridizing the DNA molecule under (i) with the DNA molecule under (ii) and of elongating the two molecules so as to generate a double stranded DNA molecule encoding a promoter 1.11, a translational initiator 1.12, a targeting sequence 2.11 for endosomial addressing fused in-frame with the tumor neoantigen or the epitope from an infectious agent 1.13 and a genetic element encoding a sequence 2.21 for the loading of the encoded tumor neoantigen or the epitope from an infectious agent for presentation to T lymphocytes.

Preferably, in the process according to the present invention, the DNA molecule (ii) is further comprising a 3'-UTR 2.31 and/or a poly A tail 2.41.

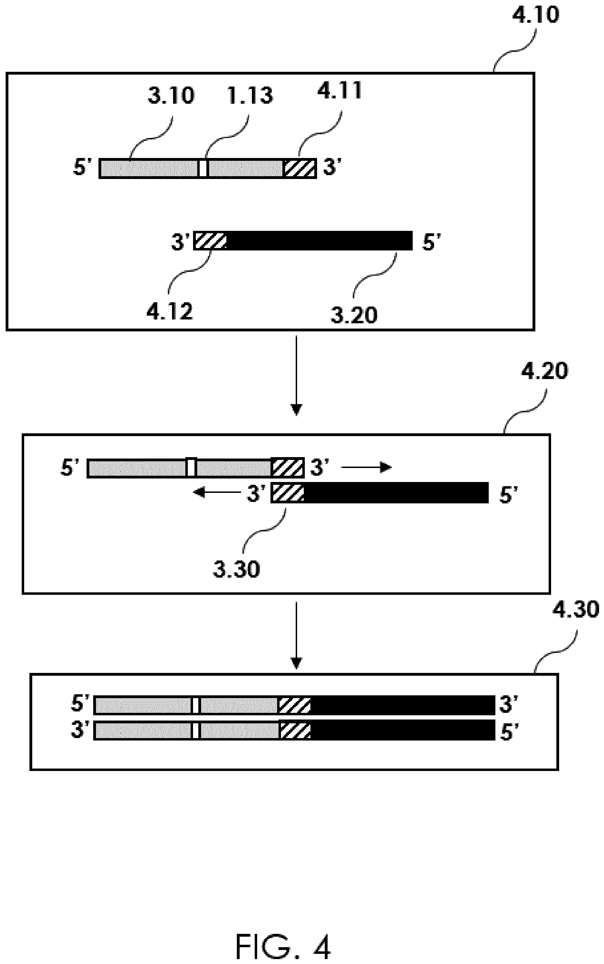

Figure 4 further illustrates in 4.10 the first single stranded DNA molecule 3.10 comprising a sequence 4.11 located in the first portion of the genetic element 2.21 being in overlap with a sequence 4.12 located in the second portion of the genetic element 2.21 of the second single stranded DNA molecule 3.20. The first single stranded DNA molecule 3.10 comprises a segment for the tumor neoantigen or the epitope from an infectious agent 1.13. The first single stranded DNA molecule 3.10 hybridized through an overlap 3.30 with the second single stranded DNA molecule 3.20 (see 4.20 in Fig. 4). Then, an elongation starting from the overlap region 3.30 allows the generation of the double stranded DNA molecule 4.30. Preferably, the elongation is an enzymatic step performed by a DNA polymerase using both single stranded DNA molecule 3.10 and 3.20 as template.

Advantageously, the present invention also relates to a pool of the DNA molecules according to the present invention or the pool obtainable by the process according to the present invention, wherein the pool comprises a plurality of different tumor neoantigens or of epitopes from an infectious agent. Preferably, each tumor neoantigen of the plurality of tumor neoantigens is a specific-tumor antigen identified in a tumor of a patient. Advantageously, the molecule 3.20 is identical and complementary to the pool of the molecules 3.10.

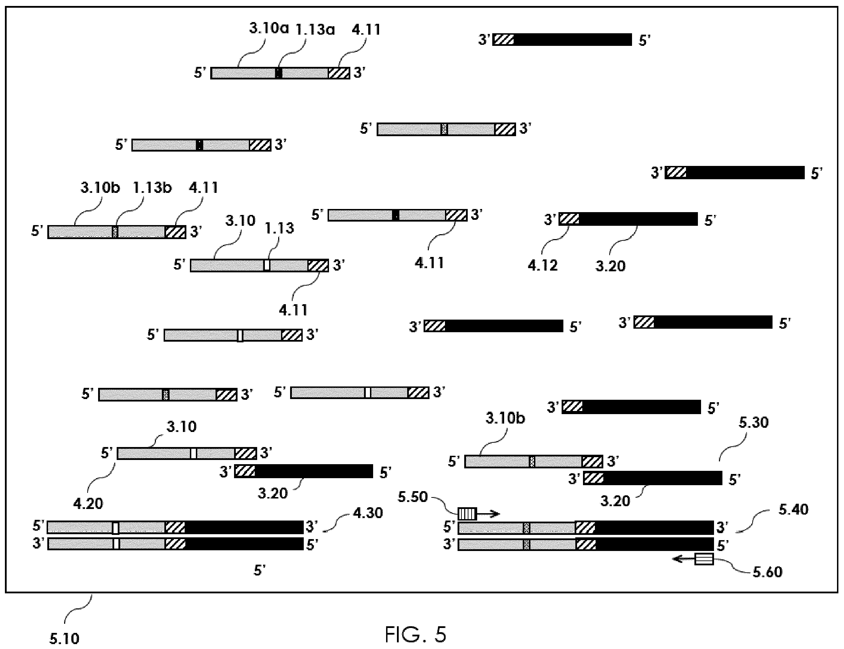

Figure 5 illustrates a pool 5.10 of DNA molecules according to the present invention or the pool obtainable by the process according to the present invention. Advantageously, the pool comprises the first single stranded DNA molecule 3.10 comprising a sequence 4.11 located in the first portion of the genetic element 2.21 being in overlap with a sequence 4.12 located in the second portion of the genetic element 2.21 of the second single stranded DNA molecule 3.20. The first single stranded DNA molecule 3.10 possesses a sequence encoding for the tumor neoantigen or the epitope from an infectious agent 1.13. Preferably, the pool also comprises a plurality of different first single stranded DNA molecules, e.g. 3.10a and 3.10b, possessing each specific sequence encoding for a second and a third specific tumor neoantigens 1.13a and 1.13b (or for a second and a third epitopes from an infectious agent), respectively. Preferably, the amino acid sequences of the tumor neoantigens encoded by the sequences 1.13, 1.13a and 1.13b are each different by at least one amino acid to the corresponding 'reference' sequence or said amino acid sequences are de novo amino acid sequences encoded from open reading frames present only in the tumor of the patient and not in normal cells, i.e. peripheral blood mononuclear cell (PBMC), of the patient. The plurality of different first single stranded DNA molecules 3.10a and 3.10b also comprises a sequence 4.11 located in the first portion of the genetic element 2.21 being in overlap with a sequence 4.12 located in the second portion of the genetic element 2.21 of the second single stranded DNA molecule 3.20. The pool may also comprise a plurality of hybridized molecules between a first single stranded DNA molecule, e.g. 3.10, 3.10a and/or 3.10b, and a second single stranded DNA molecules 3.20, e.g. an hybridized molecule 4.20 between the first single stranded DNA molecule 3.10 and the second single stranded DNA molecule 3.20, and an hybridized molecule 5.30 between the first single stranded DNA molecule 3.10b and the second single stranded DNA molecule 3.20. Advantageously, the pool also comprises a plurality of double stranded DNA molecules, wherein each double stranded DNA molecule comprises a portion of sequence corresponding to the first single stranded DNA molecule, e.g. 3.10, 3.10a or 3.10b, together with its complementary sequence and a second portion corresponding to the second single stranded DNA molecule 3.20 together with its complementary sequence. In figure 5, a double stranded DNA molecule 4.30 comprising the first single stranded DNA molecule 3.10 and the second single stranded DNA molecule 3.20 and a double stranded DNA molecule 5.40 comprising the first single stranded DNA molecule 3.10b and the second single stranded DNA molecule 3.20 are illustrated. Preferably, in the pool according to the present invention, a plurality of the DNA molecules of the pool have the same promoter for the transcription of the tumor neoantigen, or the epitope from an infectious agent, in RNA.

According to the present invention, the corresponding 'reference' sequence corresponds to the predicted peptide sequence of DNA sequenced data originating from normal cells of the patient, i.e. PBMC.

More preferably, in the pool according to the present invention, a plurality of the DNA molecules of the said pool have the same genetic element encoding a sequence for endosomial targeting of the tumor neoantigen or of the epitope from an infectious agent and/or the same translation enhancer and/or the same genetic element encoding a sequence for the loading of the encoded tumor neoantigen or the encoded epitope from an infectious agent for presentation to T lymphocytes and/or the same 3'-UTR.

Advantageously, the pool according to the present invention, comprises a plurality of second single stranded DNA molecule 3.20, wherein each second single stranded DNA molecule 3.20 of the plurality of second single stranded DNA molecule 3.20 has the same DNA sequence.

Favorably, each second single stranded DNA molecule 3.20 of the plurality of second single stranded DNA molecule 3.20 is defined by a sequence having an identity of at least 95% towards SEQ ID NO :8 over its whole sequence length.

Preferentially, the pool according to the present invention, comprises a plurality of first single stranded DNA molecule comprising a plurality of different tumor neoantigens or a plurality of different epitopes from an infectious agent.

Advantageously, since the overlap sequence 3.30 between the first single stranded DNA molecule 3.10 and the second single stranded DNA molecule 3.20 is a continuous sequence in a constant sequence region, it allows for a generation of a pool of double stranded DNA molecules by elongation of the two partially overlapping and paired strands 3.10 and 3.20.

Advantageously, the first single stranded DNA molecules 3.10, 3.10a and 3.10b are DNA molecules having each a sequence which is different between each other only for the segment for the tumor neoantigen, or the epitope from an infectious agent, 1.13 that can encode a plurality of different tumor neoantigens or a plurality of different epitopes from an infectious agent, i.e. a first specific tumor neoantigen 1.13, a second specific tumor neoantigen 1.13a and/or a third specific tumor neoantigen 1.13b. Besides the segment for the tumor neoantigen or the epitope from an infectious agent 1.13, the other segments of each first single stranded DNA molecule 3.10 of the plurality of first single stranded DNA molecule are substantially identical, e.g. the promoter 1.11 for the transcription in RNA, the segment 1.12 for its translation and the targeting sequence 2.11 for endosomial addressing and the first portion of the genetic element 2.21 encoding a sequence for the loading of the encoded tumor neoantigen or the epitope from an infectious agent for presentation to T lymphocytes.

Advantageously, the segment encoding for the tumor neoantigen, i.e. 1.13, 1.13a or 1.13b, generates a tumor neoantigen having a qualitative difference in the amino acid sequence as compared to the corresponding peptides and/or a novel amino acid sequence encoded from an open reading frame wherein said open reading frame is only present in the tumor and not in normal cells of the patient. Preferably, each tumor neoantigen of a plurality of different tumor neoantigens is defined by an amino acid sequence different by at least one amino acid between each other.

According to the present invention, the pool of double stranded DNA molecules counts at least 10, preferably at least 20, more preferably at least 40, even more preferably at least 60, favorably at least 100 different double-stranded DNA molecules encoding each a different tumor neoantigen (or a different epitope from an infectious agent), wherein a double-stranded DNA molecule is different from another double-stranded DNA molecule of the pool of double-stranded DNA molecules if the double-stranded DNA molecule is defined by a sequence encoding for a tumor neoantigen having an amino acid sequence different by at least one amino acid from the amino acid sequence of another neoantigen encoded by said another double-stranded DNA molecule. Of course, the pool may contain multiple copies of the same double-stranded DNA molecule.

Advantageously, each tumor neoantigen of a plurality of different tumor neoantigens is defined by an amino acid sequence having an identical mutation as compared to the amino acid sequence of its corresponding 'reference' sequence or is defined by a de novo amino acid sequence encoded from de novo open reading frames only present in the DNA of the tumor of the patient and not present in the DNA from normal cells of the patient, i.e. PBMC, wherein the identical mutation is located at a different position within the amino acid sequence for each neoantigen of the plurality of different tumor neoantigens. For example, for a first tumor neoantigen, a first mutation is located substantially in the middle of the amino acid sequence of the first tumor neoantigen, while for a second tumor neoantigen, the identical first mutation is located substantially in the first half or in the first quarter or in the second half or in the last quarter of the amino acid sequence of the second tumor neoantigen, wherein the first amino acid of the tumor neoantigen correspond to the first 5' codon of the segment for the tumor neoantigen 1.13.

Preferably, the pool of double stranded DNA molecules is amplified by PCR amplification using a forward primer 5.50 and a reverse primer 5.60. Preferably, the reverse primer is an oligonucleotide poly T that can extend to the 3' end of the DNA molecule, such as SEQ ID NO :79 or SEQ ID NO :80, according to the present invention.

Preferably, the generation of a pool of double stranded DNA molecules and the amplification of the pool of double stranded DNA molecules are performed using the same tube, so that the same reactants (primer pairs, polymerase, dNTPs, reaction medium) are used, firstly to elongate the two strands, each strand acting as a template for the elongation of the other, then to amplify them in a concomitant or subsequent manner, with a separation of the two elongated strands, the base pairing of the specific primers (5.50, 5.60) at a lower temperature, then the elongation at an higher temperature. In an alternative, the primers are added to the reaction medium after that the first elongation, i.e. the first elongation step, has been completed.

Preferably, the pool of double stranded DNA molecules also comprises segments recognized by a RNA-dependent polymerase, such as the nonstructural proteins of the alphavirus nsP1-4. Indeed, when the pool of double stranded DNA molecules is used as a template for in vitro transcription of the DNA molecules into RNA molecules to produce a RNA vaccine it is useful that the produced mRNAs are able of trans-amplification, such as the trans-amplification based on the encoded RdRp. Such RNA trans-amplification permits to reduce the dose of mRNA vaccine administrated to the patient.

Preferably, the present invention also relates to a process for the generation of a RNA vaccine towards a cancer affecting a patient comprising the steps of generating the DNA molecule according to the present invention or the pool according to the present invention and of performing in vitro transcription of the DNA molecule(s) into RNA molecule(s).

Preferably, the process for the generation of a RNA vaccine according to the present invention further comprising the step of formulating the RNA molecule(s) into a vaccine.

Advantageously, the present invention also relates to the vaccine obtainable by the process for the generation of a RNA vaccine according to the present invention.

Favorably, the present invention also relates to the vaccine obtainable according to the present invention for use in the treatment of a cancer affecting a patient.

### Examples.-

### Example 1: Identification of tumor neoantigens of a tumor of a patient.

The inventors performed a whole genome sequencing of DNA from a tumor sample of a patient and of DNA from peripheral blood mononuclear cell (PBMC) of the patient. An RNASeq of the tumor sample was also performed. An identification of the tumor neoantigens was realized based on the open reading frames (ORFs) for which mRNAs are identified from the RNASeq of the tumor, wherein mRNAs have a predicted peptide sequences having at least one identified difference as compared to a reference peptide sequence. The reference peptide sequence being the predicted peptide sequence originated from the open reading frames identified from the whole genome sequencing data from the DNA of PBMC of the patient. The identified difference can be the result of a point mutation with a different amino acid as compared to the reference peptide sequence or a translocation, a deletion and/or an indel, or a fusion. A detailed protocol to identify tumor neoantigens of the tumor of the patient can be found in the patent application EP21196366.5

### Example 2: Identification of the amino acid sequences and DNA sequences of the neoantigens.

The inventors identify a neoantigen peptide sequence of 27 amino acids, wherein the identified difference as compared to the reference peptide sequence is located substantially in the middle of the 27 amino acid sequence length of the neoantigen peptide sequence.

The neoantigen peptide sequence is converted into a nucleic acid sequence by means of online tools. Such tools optimize the use of codon for an optimal expression in humans and reduces the formation of secondary structure of the RNA, as well as unwanted immune response, or increases the desired immune responses when the nucleic acid sequence is used as a template in a RNA vaccine.

### Example 3: Synthesis of a pool of DNA molecules according to the present invention comprising a plurality of tumor neoantigens.

The inventors synthetized single stranded DNA molecules in pool using a silicon-based DNA writing technology (see Twist Oligo Pools technology from TWIST Bioscience) comprising the DNA sequence of a plurality of tumor neoantigens obtained in the example 2. In the pool according to the present invention, a plurality of second single stranded DNA molecules is synthetized having each substantially the same sequence SEQ ID NO :8 with a length of 297 nucleotides. A plurality of 60 different first single stranded DNA molecules is also synthetized in the pool, wherein the 60 different first single stranded DNA molecules having substantially the sequences SEQ ID NO :13 to SEQ ID NO :72, wherein each sequence SEQ ID NO :13 to SEQ ID NO :72 is a sequence with a length of 291 nucleotides. The second single stranded DNA molecule being in antisense can hybridize with each first single stranded DNA molecule with an overlap having a sequence corresponding to the nucleotides 30 to 52 (sequence for sense/antisense pairing) of SEQ ID NO :5.

### Example 4: A DNA molecule according to the present invention

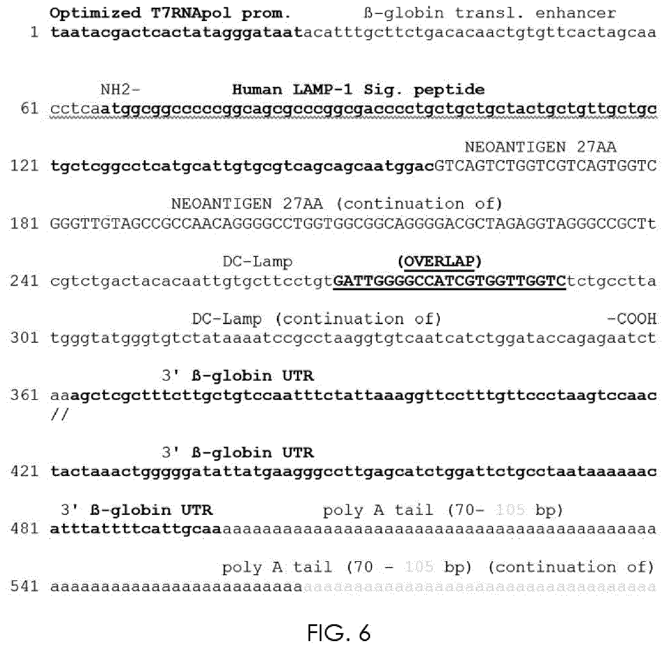

An example of a sequence of a DNA molecule obtained according to the present invention is illustrated in Figure 6 showing a DNA sequence of 600 nucleotides. Starting from the first nucleotide 1, the DNA molecule comprises an optimized T7 RNA polymerase promoter segment (lower case letter in bold in the sequence of the DNA molecule in Figure 6) followed by a sequence for the beta-globin translation enhancer segment (lower case letter). The next segment is a segment encoding for the human LAMP-1 signal peptide (lower case letter in bold) followed by a segment encoding the tumor neoantigen of 27 amino acids (upper case letter). The next segment is a segment encoding for the DC-Lamp (lower case letter). Within the DC-Lamp sequence, an overlap sequence is present (highlighted upper case letter) representing the overlap sequence between the first and second single stranded DNA molecules. After the DC-Lamp sequence, a 3' UTR beta-globin segment (beta-globin UTR) is present for translation and stabilization optimization (lower case letter in bold) followed by a poly A tail of either 70 nucleotides up to 105 nucleotides.

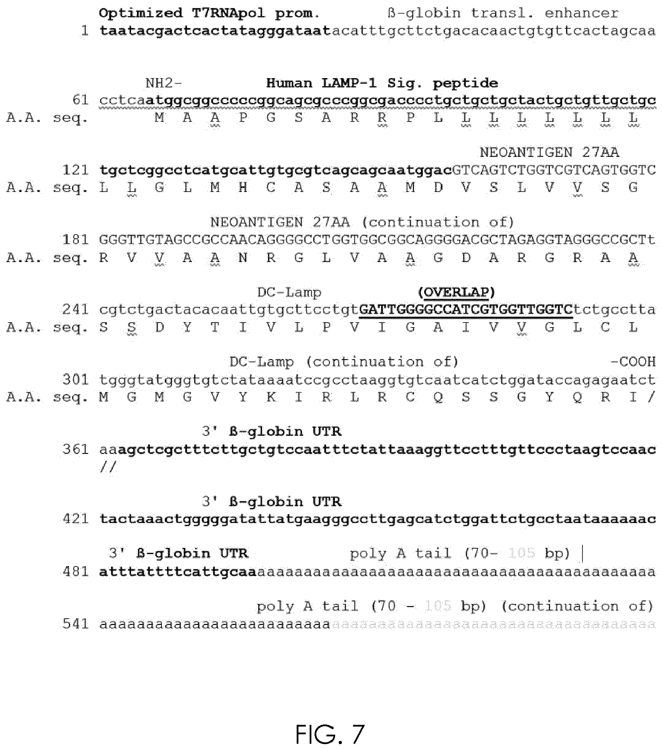

Figure 7 illustrates the same sequence of DNA molecule, wherein the amino acid sequence of the encoded part of the DNA sequence is juxtaposed to the DNA sequence.

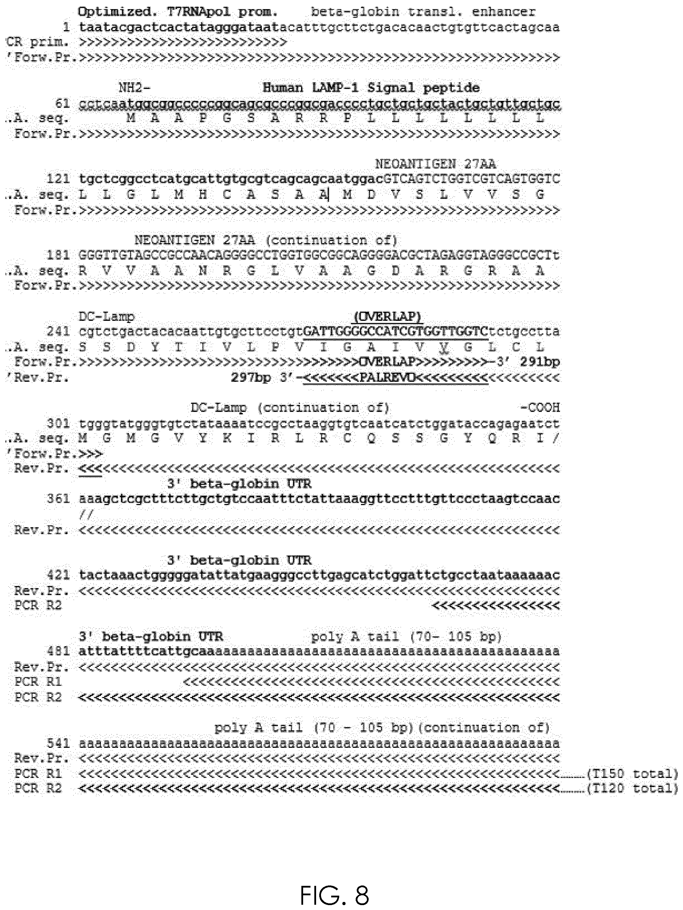

Figure 8 is similar to Figure 7 where the 5' to 3' sequence of the first single stranded DNA molecule is represented (Forward Pr.) while the 5' to 3' sequence of the second single stranded DNA molecule, being in antisense with the first single stranded DNA molecule, is also represented (Rev. Pr.). The forward (PCR prim.) and reverse primers (gc-T150) used in a subsequent PCR amplification are also represented.

### Example 5: Creation of a pool of double-stranded DNA molecules

The hybridized DNA molecules between the first and second single stranded DNA molecules are obtained in the Example 3. From these hybridized DNA molecules, an elongation of each strand using a DNA polymerase allows the production of double-stranded DNA molecules at a femtomolar concentration level.

### Example 6: Amplification of a pool of double-stranded DNA molecules

To be able to produce a sufficiently large amount of double-stranded DNA molecules needed for subsequent in vitro transcription allowing for the production of RNA molecules that will be incorporated into the RNA vaccine according to the present invention, we have amplified the pool of double-stranded DNA molecules obtained in the Example 5. To do so, we performed 13 cycles of PCR amplification of the double-stranded DNA molecules using a forward primer having a sequence corresponding to SEQ ID NO :7 and a reverse primer having a sequence 5'(T100)-GC -3' corresponding to SEQ ID NO :79.

The PCR reaction components are :
- 5× KAPA HiFi Fidelity Buffer (1×),
- 10 mM each dNTP Mix,
- 10 µM Forward Primer,
- 10 µM Reverse Primer,
- the pool of DNA molecules obtained in the Example 4 (20ng/ul),
- KAPA HiFi HotStart DNA polymerase (1 U/ul) and,
- PCR grade water.

The PCR reaction conditions are:
- Initialization denaturation (3 min at 95°C)
- Denaturation (20 sec at 98°C)
- Annealing (15 sec)
- Extension (15 sec at 72°C)
- Final extension (1 min at 72°C).

### Example 7: In vitro transcription of the pool of double-stranded DNA molecules

The amplified pool of double-stranded DNA molecules obtained in the Example 6 is in vitro transcribed to produce a pool of corresponding RNA molecules.

## Claims

1. A synthetic DNA molecule comprising one segment encoding a tumor neoantigen, or an epitope from an infectious agent, under the control of a promoter for the transcription into a corresponding RNA molecule, and a segment for the translation of the said translated RNA molecule into a peptide.

2. The DNA molecule of claim 1 further comprising a segment encoding a sequence for stabilizing and/or targeting and/or trafficking the tumor neoantigen or the epitope from an infectious agent in a vesicular region and/or a sequence for addressing the encoded tumor neoantigen or the epitope from an infectious agent to MHC molecules.

3. The DNA molecule according to any one of the preceding claims further comprising a segment encoding a translation enhancer and/or a 3'-UTR and/or a 3' Poly A tail segment(s) and/or a 5'-UTR.

4. The DNA molecule according to any one of the preceding claims being double stranded and having a size comprised between 100 nucleotides per strand and about 2500 nucleotides per strand, preferably between 300 nucleotides per strand and about 800 nucleotides per strand, more preferably between about 400 nucleotides per strand and about 700 nucleotides per strand, still more preferably between about 500 (or 550) nucleotides per strand and about 650 (or 600) nucleotides per strand.

5. A DNA molecule comprising (i) a segment encoding a (second) portion of a peptide for addressing an antigen to MHC molecules, (ii) a segment for increasing the stability of a transcribed RNA or for increasing the translation of the transcribed RNA into a protein, and (iii) a segment encoding a poly A tail.

6. The DNA molecule of claim 5 being in antisense, preferably for base pairing with a sense DNA molecule according to any one of the preceding claims 1 to 4.

7. A process for the production of the DNA molecule according to any one of the preceding claims 1 to 4, comprising the steps of performing the chemical synthesis of the DNA molecule encoding the tumor neoantigen, or the epitope from an infectious agent, under the control of a promoter.

8. The process of claim 7 comprising the steps of performing the chemical synthesis of (i) a first single stranded DNA molecule comprising the promoter for the transcription in RNA, the segment for its translation and a targeting sequence for endosomial addressing fused in-frame with the tumor neoantigen, or the epitope from an infectious agent, and a first portion of a genetic element encoding the sequence for the loading of the encoded tumor neoantigen, or the epitope from an infectious agent, for presentation to T lymphocytes, and of (ii) a second single-stranded DNA molecule encoding a second portion of a genetic element encoding the sequence for the loading of the encoded tumor neoantigen, or the epitope from an infectious agent, for presentation to T lymphocytes, wherein the second DNA molecule is antisense and wherein the said first and second portions form the complete sequence for the loading of the tumor neoantigen or the epitope from an infectious agent for presentation to T lymphocytes and wherein the said first and second portions present an overlap (of at least 15 consecutive nucleotides) allowing a specific base pairing, the said process further comprising the step of hybridizing the DNA molecule under (i) with the DNA molecule under (ii) and of elongating the two molecules so as to generate a double stranded DNA molecule encoding a promoter, the translational enhancer, the targeting sequence for endosomial addressing fused in-frame with the tumor neoantigen, or the epitope from an infectious agent, and a genetic element encoding the sequence for the loading of the encoded tumor neoantigen, or the epitope from an infectious agent, for presentation to T lymphocytes.

9. The process of claim 7 or 8, wherein the DNA molecule (ii) is further comprising a 3'-UTR and/or a poly A tail.

10. A pool of the DNA molecules according to any one of the preceding claims 1 to 5 or the pool obtainable by the process according to the claims 7 to 9, the said pool comprising a plurality of different tumor neoantigens or a plurality of different epitopes from an infectious agent.

11. The pool of claim 10, wherein a plurality of the DNA molecules of the said pool have the same promoter for the transcription of the tumor neoantigen, or the epitope from an infectious agent, in RNA.

12. The pool of claim 10 or 11, wherein a plurality of the DNA molecules of the said pool have the same genetic element encoding a sequence for endosomial targeting of the tumor neoantigen, or the epitope from an infectious agent, and/or the same translation enhancer and/or the same genetic element encoding a sequence for the loading of the encoded tumor neoantigen, or the epitope from an infectious agent, for presentation to T lymphocytes and/or the same 3'-UTR.

13. A process for the generation of a RNA vaccine towards a cancer affecting a patient comprising the steps of generating the DNA molecule according to any one of the preceding claims 1 to 4 or the pool according to any one of the claims 10 to 12 and of performing in vitro transcription of the DNA molecule(s) into RNA molecule(s).

14. The process of claim 13 further comprising the step of formulating the RNA molecule(s) into a vaccine).

15. A vaccine obtainable by the process of claim 14 for use in the treatment of a cancer, or an infectious disease, affecting a patient.
